# EUROPEAN PATENT SPECIFICATION

(11) **EP 0 532 629 B1**
(45) Date of publication and mention of the grant of the patent: **06.11.1996**
(21) Application number: 91911167.4
(22) Date of filing: 15.05.1991
(51) Int. Cl.: A61K 31/445, C07D 211/18, C07D 211/22, C07D 211/32

(54) **1-PIPERIDINYL ALKANOYLARYLSULFONAMIDE DERIVATIVES**
DERIVATE DES 1-PIPERIDINYL-ALKANOYLARYLSULFONAMIDS
DERIVES D'ALCANOYLARYLSULFONAMIDE DE PIPERIDINYLE-1

(30) Priority: 07.06.1990 US 534784
(43) Date of publication of application: 24.03.1993
(73) Proprietor: MERRELL PHARMACEUTICALS INC., Cincinnati, Ohio 45215-6300 (US)
(72) Inventor: CARR, Albert, A., Cincinnati, OH 45237 (US); DAGE, Richard, C., Cincinnati, OH 45242 (US); NIEDUZAK, Thaddeus, R., Golf Manor, OH 45237 (US); KOERNER, John, E., Cincinnati, OH 45236 (US); LI, Tung, Cincinnati, OH 45237 (US)
(74) Representative: Tauchner, Paul, Dr.
(86) International application number: US9103323
(87) International publication number: WO9118603

(56) References cited:
- EP-A- 0 202 164
- EP-A- 0 304 888
- EP-A- 0 320 983
- EP-A- 0 437 790
- US-A- 4 665 067
- US-A- 4 876 262
- A. BURGER, "A guide to the chemical basis of drug design", John Wiley & Sons, p. 15, 1984.

## Description

The present invention is directed to a new class of 1-piperidinyl alkanoylarylsulfonamide derivatives which are useful as Class III antiarrhythmic agents. Another aspect of the invention is directed to a method for treating cardiac arrhythmias. An additional aspect of the invention is directed to pharmaceutical compositions containing these compounds.

In EP-A 0 304 888, EP-A 0 202 164 and EP-A 0 437 790 pharmacologically active piperidinyl compounds are disclosed.

In accordance with the present invention a new class of antiarrhythmic agents have been discovered which can be described by the following formula: 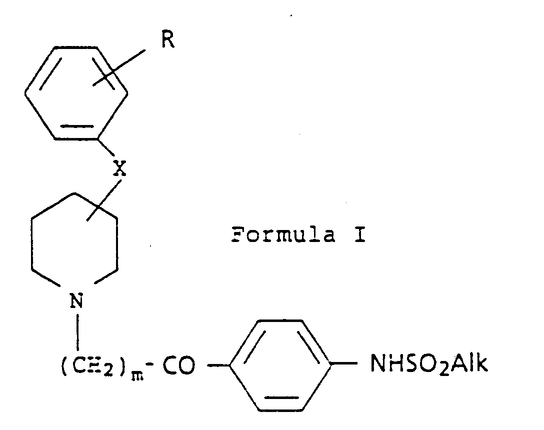 in which R is represented by hydrogen, halogen, C₁₋₄ alkyl, C₁₋₄ alkoxy, CF₃, SR₁, NHC(O)R₂, NH₂, or OH wherein the phenyl ring may contain upto 3 non hydrogen substituents; R₁ is hydrogen or C₁₋₄ alkyl; R₂ is C₁₋₄ alkyl; X is represented by CO or CHOH; m is an integer from 1-3; and Alk is a C₁₋₄ alkyl; and the pharmaceutically acceptable addition salts thereof.

As used in this application:
a) the term "halogen" refers to a fluorine, chlorine, or bromine atom;
b) the terms "lower alkyl group and C₁₋₄ alkyl" refer to a branched or straight chained alkyl group containing from 1-4 carbon atoms, such as methyl, ethyl, n-propyl, isopropyl, n-butyl, isobutyl, etc.;
c) the terms "lower alkoxy group and C₁₋₄ alkoxy" refer to a straight or branched alkoxy group containing from 1-4 carbon atoms, such as methoxy, ethoxy, n-propoxy, isopropoxy, n-butoxy, isobutoxy, etc.;
d) the term "pharmaceutically acceptable addition salt refers to either an acid addition salt or a basic addition salt;
e) the term "CO" refers to a carbonyl group having the following structure: 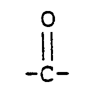
f) the term "CHOH" refers to a hydroxymethylene group;
g) the term "ketal" refers to the following substituent:

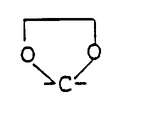

Preferred compounds include those wherein m is 1 or 2 and wherein Alk is methyl.

The expression "pharmaceutically acceptable acid addition salts" is intended to apply to any non-toxic organic or inorganic acid addition salt of the base compounds represented by Formula I or any of its intermediates. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic, p-toluenesulfonic acid and sulfonic acids such as methanesulfonic acid and 2-hydroxyethane sulfonic acid. Either the mono- or di-acid salts may be formed, and such salts may exist in either a hydrated or substantially anhydrous form.

The expression "pharmaceutically acceptable basic addition salts" is intended to apply to any non-toxic organic or inorganic basic addition salts of the compounds represented by Formula I or any of its intermediates. Illustrative bases which form suitable salts include alkali metal or alkaline-earth metal hydroxides such as sodium, or potassium, and aliphatic or alicyclic amines, such as methylamine, dimethylamine, or trimethylamine. Either the mono- or di-basic salts may be formed with those compounds.

Some of the compounds of Formula I exist as optical isomers. Any reference in this application to one of the compounds represented by Formula I is meant to encompass either a specific optical isomer or a mixture of optical isomers. The specific optical isomers can be separated and recovered by techniques known in the art such as chromatography on chiral stationary phases, resolution via chiral salt formation and subsequent separation by selective crystallization or ester formation from a chiral acid followed by separation of the resultant diasterimoeric esters and hydrolysis to the desired enantiomer.

The X substituent may be bonded to either the 3-position or the 4-position of the piperidinyl ring. The phenyl ring adjacent to the 3- or 4-position of the piperidinyl ring may be optionally substituted. It is possible for this phenyl ring to contain up to 3 non-hydrogen substituents. These substituents can be located at any of the ortho, meta or para positons. These substituents can be the same or different.

Illustrative Examples of compounds encompassed by the present invention include:
N-[4-[[4-(2,3-dimethoxybenzoyl)-1-piperidinyl]acetyl]phenyl]methanesulfonamide,
N-[4-[[4-(3,4-difluorobenzoyl)-1-piperidinyl]acetyl]phenyl]methanesulfonamide,
N-[4-[[4-(2,4,6-trimethylbenzoyl)-1-piperidinyl]acetyl]phenyl]methanesulfonamide,
N-[4-[[4-[4-(methylthio)benzoyl]-1-piperidinyl]acetyl]phenyl]methanesulfonamide,
N-[4-[[4-(2-ethoxybenzoyl)-1-piperidinyl]acetyl]phenyl]methanesulfonamide,
N-[4-[3-[4-[(3,4-difluorobenzoyl)-1-piperidinyl]-1-oxopropyl]phenyl]methansulfonamide,
N-[4-[[4-[(2,3-dimethoxyphenyl)hydroxymethyl]-1-piperidinyl]acetyl]phenyl]methanesulfonamide,
N-[4-[[4-[(4-fluorophenyl)hydroxymethyl]-1-piperidinyl]acetyl]phenyl]methanesulfonamide,
N-[4-[4-[4-(4-fluorobenzoyl)-1-piperidinyl]-1-oxobutyl]phenyl]methanesulfonamide,
N-[4-[[4-[(3,4-difluorophenyl)hydroxymethyl]-1-piperidinyl]acetyl]phenyl]methansulfonamide,
N-[4-[3-[4-[(3,4-difluorophenyl)hydroxymethyl]-1-piperidinyl]-1-oxopropyl]phenyl]methanesulfonamide,
N-[4-[3-[4-(4-fluorobenzoyl)-1-piperidinyl]-1-oxopropyl]-phenyl]methanesulfonamide,
N-[4-[[(4-fluorobenzoyl)-1-piperidinyl]-acetyl]phenyl]methanesulfonamide,
N-[4-[[(4-chlorobenzoyl)-1-piperidinyl]acetyl]-phenyl]methanesulfonamide,
N-[4-[3-[(4-chlorobenzoyl)-1-piperidinyl]-1-oxopropyl]phenyl]methanesulfonamide,
N-[4-[3-[4-(4-fluorobenzoyl)-1-piperidinyl]1-oxopropyl]phenyl]methanesulfonamide.

It is preferred for the compounds of Formula I to be 4-piperidino derivatives. It is preferred that R be represented by a 4-fluoro or a 3,4-difluorosubstituent. It is also preferred for m to be represented by 2.

The compounds of Formula I can be prepared using techniques which are analgously known in the art. One method for preparing these compounds is illustrated below in Reaction Scheme I:

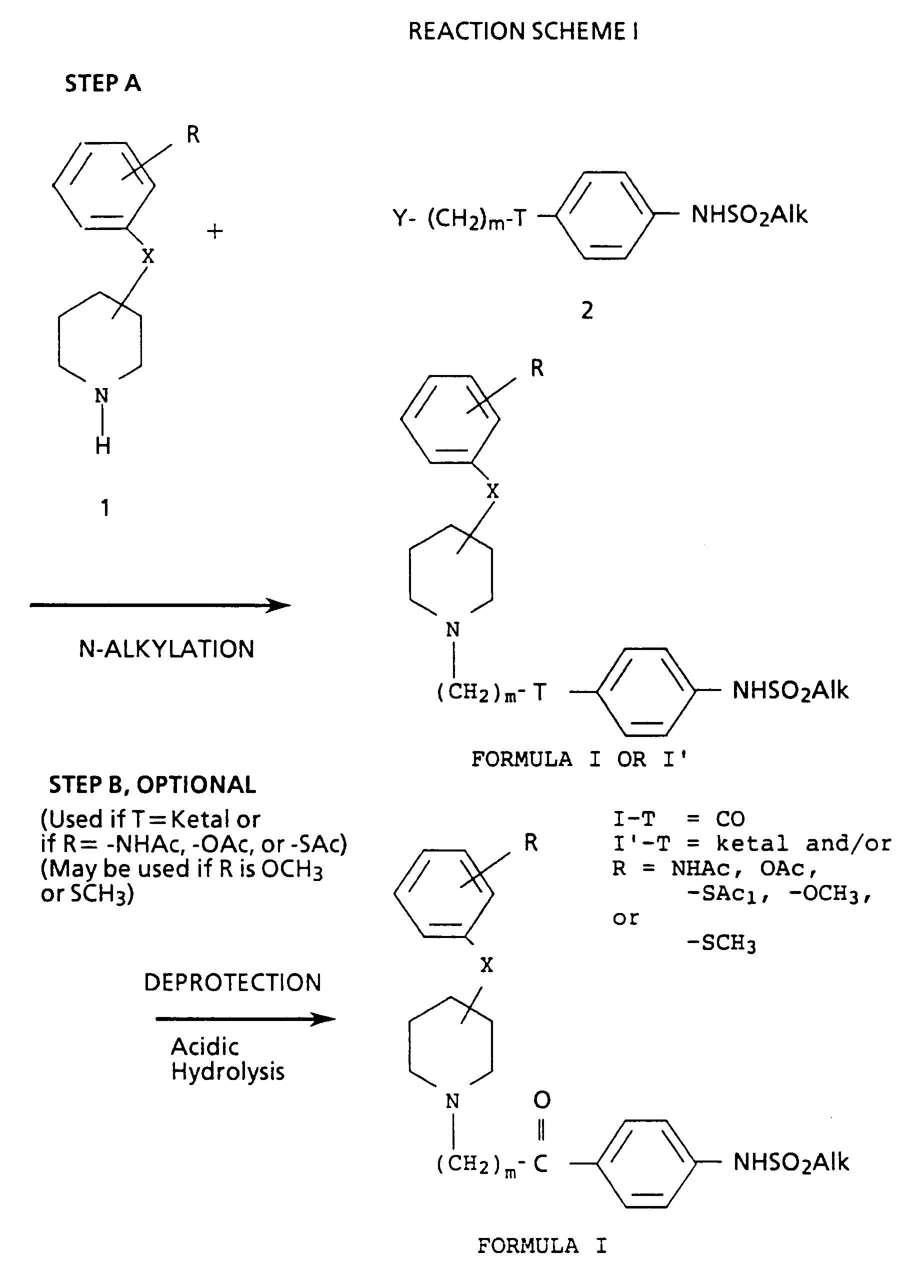

In Step A of Reaction Scheme I, an N-alkylation reaction is carried out between a 3-or 4-substituted piperidine as described by structure 1, in which R and X are as in Formula I, and an N-alkylarylsulfonamide as described by structure 2, in which m and Alk are as in Formula I, Y is a leaving group such as a halogen atom or tosylate, and T is either CO or a ketal function, with the proviso that when R is to be OH, SH, or NH₂, then the corresponding acetyl derivative is utilized (i.e., OAc, NHAc, or SAc). If T is a ketal function or if R is an acetyl derivative, then it is necessary to carry out the optional Deprotection Reaction of Step B which converts any ketal function into a carbonyl group as is depicted and removes any acetyl function which leaves an OH, SH, or NH₂ substituent on the molecule.

The appropriate piperidinyl compound to utilize is one in which X and R are represented by the same substituent as is desired in the final product of Formula I or R is one of the acetyl functions identified above. X should be bonded to the 3- or 4- position as is desired in the final product. The appropriate N-alkylarylsulfonamide is one in which Alk and m are represented by the same substituent as is desired in the final product. The particular leaving group that Y represents is not critical since it is not retained in the final product. If m is represented 1 or 2, then T should be represented by CO. If m is to be represented by 3, then T should be represented by a ketal which serves to prevent cyclopropane formation.

For example if the desired product is, N-[4-[3-[4-(4-fluorobenzoyl)-1-piperidinyl]-1-oxopropyl]phenyl]-methanesulfonamide, then the proper reactants are 4-fluorophenyl-4-piperidinyl methanone and N-[4-(3-chloro-1-oxopropyl)phenyl] methanesulfonamide. N-[4-[4-[4-(4-fluorobenzoyl)-1-piperidinyl]-1-oxobutyl]phenyl]-methanesulfonamide can be produced by reacting 4-fluorophenyl-4-piperidinyl methanone and 2-(3-chloropropyl)-2-(4-methanesulfonamidophenyl) 1,3-dioxolane.

The N-alkylation can be carried out using techniques known in the art. Approximately equivalent amounts of the piperidinyl compound of structure 1 and the alkanoylarylsulfonamide of structure 2 are contacted in a solvent such as aqueous tetrahydrofuran, n-butanol, toluene or dimethylformamide, etc. The reaction is carried out in the presence of at least one equivalent of a weak base such as potassium bicarbonate, potassium carbonate or sodium bicarbonate. If desired the N-alkylation can be carried out in the presence of a catalytic amount of potassium iodide. The reaction is typically carried out at a temperature range of from room temperature to reflux for a period of time ranging from 1 hour to 5 days.

The crude product of Formula I or I' can be recovered from the reaction medium and purified by techniques known in the art. For example, the crude compounds can be recovered by organic solvent extraction in the presence of water. Suitable extraction solvents include ethyl acetate, dichloromethane or chloroform. The resulting crude material can then be purified by recrystallization from a solvent system such as methanol, methanol/butanone, isopropanol or chloroform, etc. The compounds may also be purified by chromatographic techniques such as silica gel chromatography. Suitable chromatographic sovlents include ethyl acetate, acetone, ethyl acetate/hexane, or ethyl acetate/acetone.

The Optional Deprotection Reaction of Step B can also be carried out using techniques known in the art. Typically the crude product of Formula I' in which X is CO is subjected to an acidic hydrolysis with a mineral acid such as HCL (about 0.1-.5 N in methanol). The hydrolysis is typically carried out at a temperature range of from 0 to 30°C for a period of time ranging from 0.5 to 5 hours. If X is represented by CHOH, then a mild organic acid such as tartaric acid should be utilized. Typically a slight molar excess of the organic acid is utilized in a solvent such as aqueous THF at a temperature of about 0°C for about 0.5 hours. After either hydrolysis is completed, the reaction medium is neutralized with a mild base such as sodium bicarbonate. The resulting compound of Formula I can be recovered and purifed by the same methods taught in Step A of Reaction Scheme I.

Methods for producing the N-alkylarylsulfonamides of structure 2 are known in the art. Methods for producing the 3- and 4-substituted piperidines of structure 1 are known in the art. For example, those in which X is CO may be prepared by carrying out a Friedel-Craft reaction between an appropriately substituted benzene and isonipecetic acid chloride HCl. Reaction of an appropriately substituted phenyl Grignard reagent with 3- or 4-cyano-1-trifluoroacetylpiperidine is also suitable, followed by deprotection with Na₂CO₃/methanol. Those compounds in which X is CHOH may then be produced by reducing ketones produced above. Appropriate reduction techniques include either catalytic hydrogenation or hydride reducing agents such as sodium borohydride.

These reactions are well known in the art and are illustrated in United States Patent No. 4,783,471 which is hereby incorporated by reference.

Alternatively, as illustrated below in Reaction Scheme II, the 3- and 4-substituted piperidines of structure 1 in which X is CHOH can be prepared by reacting an appropriately substituted phenyl magnesium bromide (structure 3; Grignard Reagent) with 3- or 4-pyridine carboxaldehyde, structure (4), as is known in the art, thereby producing the carbinol of structure (5). This compound is catalytically reduced as is known in the art which produces the substituted piperidines of structure 1 in which X is CHOH. Oxidation with CrO₃ will produce the corresponding ketone.

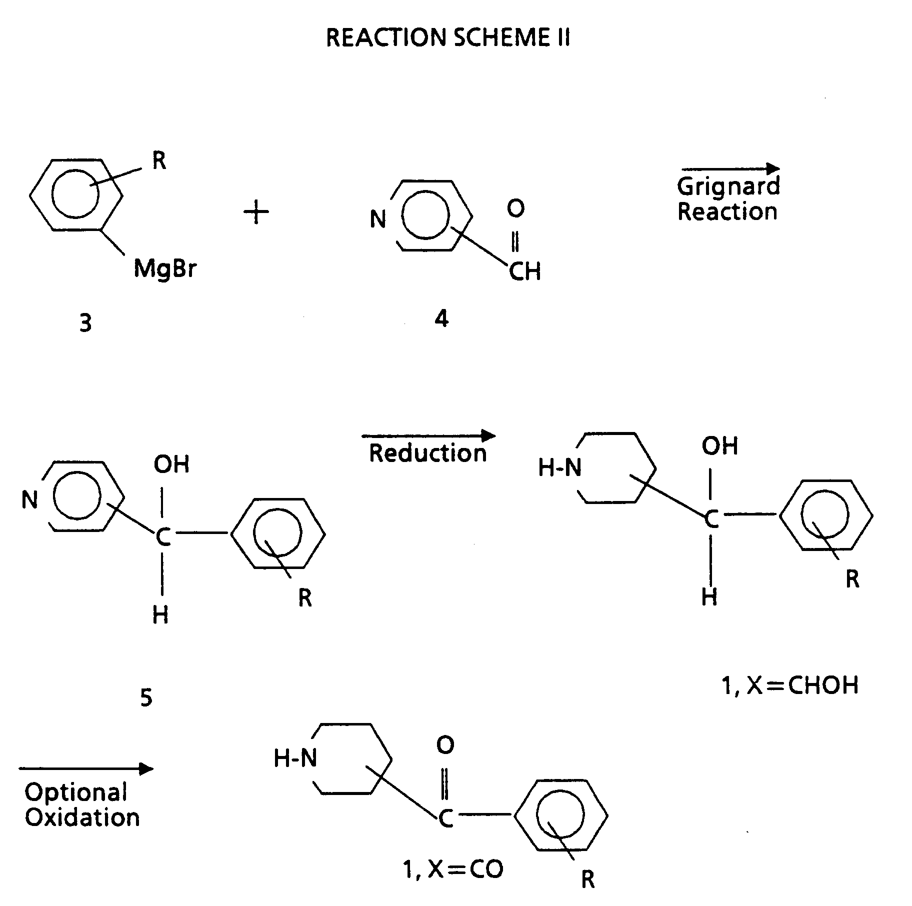

Another method for producing the 3- and 4-substituted piperidines of structure 1 in which X is CO is the following 3-step synthesis illustrated below in Reaction Scheme III. In Step A, an acylation reaction is carried out between 1-(1,1-dimethylethyl)-1,3-(or 4)-piperidinedicarboxylic acid, structure (6), and N,O-dimethylhydroxylamine hydrochloride, structure (7), in the presence of 1,1-carbonyldiimidazole. The reactants are present in an equivalent amount and the reaction is carried out at room temperature under a nitrogen atmosphere in methylene dichloride. The piperidine of structure (6) is first contacted with the 1,1-carbonyldiimidazole for about 1 hour. The hydroxylamine of structure (7) is then added and the reactants are stirred together for 1 - 12 hours. The resulting 3- or 4-(N-methoxy-N-methyl-carboxamido)-1-piperidinecarboxylic acid 1,1-dimethylethylester, structure (8) can be recovered by acidification, extraction and then purified by distillation.

In Step B, a appropriately substituted benzene derivative (or bromo-substituted benzene derivative) is contacted with n-butyl lithium at about 0°C under an argon atmosphere for about 1 hour. The reaction is then cooled to -78°C and an equivalent amount of the product of Step A is added to the reaction. Upon completion of the addition, the reaction is warmed to room temperature and stirred for approximately 3 hours. This displacement reaction produces an N-protected piperidine of structure (1) in which X is CO. It can be recovered by extraction and purified by silica gel chromatography. In Step C, the protecting group at the 1-position of the piperidine ring is removed by hydrolysis with trifluoroacetic acid as is known in the art.

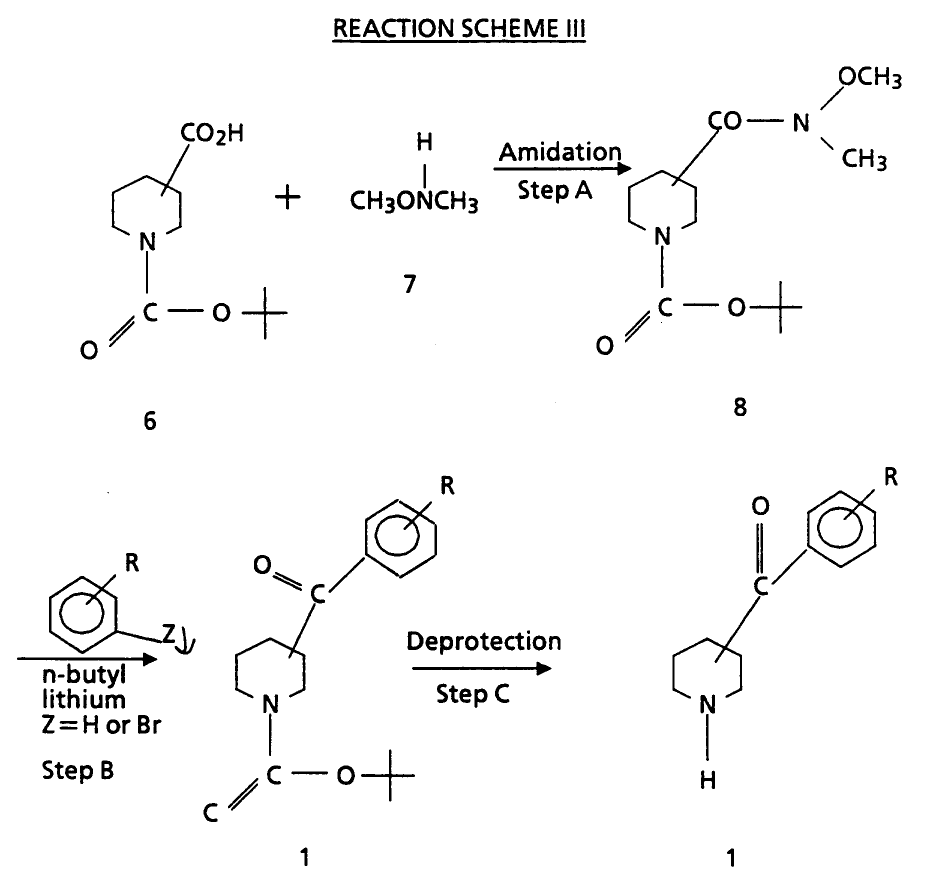

The compounds of Formula I are useful as cardiac antiarrhythmic agents. They can be administered to a patient suffering from an arrhythmic episode in order to terminate that episode and return the myocardium to a normal sinus rhythm. The compounds can also be administered on a prophylactic basis to prevent the occcurence of arrhythmic episodes.

The compounds of Formula I increase the duration of the action potential of myocardial tissue producing an increase in the refractory period of that tissue. Thus, under the classification system of Vaughan Williams these compounds exhibit a Class III antiarrhythmic activity.

One method of demonstrating the antiarrhythmic activity of these compounds is the following test protocol. This protocol demonstrates what effect a compound has upon the action potential of isolated cardiac tissue, such as a Purkinje fiber from a dog heart or a papillary muscle from a guinea pig heart.

The heart of an anesthetized mongrel dog is surgically removed and the Purkinje fibers are dissected from either of the ventricles. Alternatively, papillary muscles are removed from the right cardiac ventricle of a guinea pig. A Purkinje fiber or a papillary muscle is then placed in a tissue bath which is continuously perfused with modified Tyrode's solution. The modified Tyrode's solution has the following composition (in mM): NaCl 127.0, KCl 5.4, NaH₂PO₄ 0.5, MgCl₂ 1.0, NaHCO₃ 23.8, CaCl₂ 1.8 and glucose 11.1. A gas mixture comprised of 95% O₂ and 5% CO₂ is bubbled through the solution while it is maintained within a pH range of from 7.3-7.4.

The electrophysiology of the cardiac tissue is monitored by conventional glass microelectrodes. One microelectrode is inserted into a cell in the cardiac muscle fiber and a ground electrode is positioned in the tissue bath. A conventional oscilloscope is utilized to visualize the action potential waveforms of the cardiac cell.

The cardiac muscle fiber is electrically stimulated at a frequency of 1 Hz through a pair of platinum plates placed in the tissue bath. This stimulation is continued for approximately 1 hour in order to allow the electrophysiological characteristics of the fiber to stabilize.

After approximately 1 hour, the fiber should be exhibiting a stable action potential as demonstrated by the waveform displayed on the oscilloscope. At this point, representative control action potentials are recorded and analyzed by a computer.

After establishing a control action potential, the test compound is introduced into the Modified Tyrode's solution in a quantity such that the test compound is present within the tissue bath in a range of from 10⁻⁸ to 10⁻⁵ moles/liter. After the effect of the test compound has reached a steady state, the action potential is again recorded and analyzed in the manner described above.

The *in vivo* antiarrhythmic activity of the compounds can be demonstrated in the following manner. Mongrel dogs of either sex are anesthetized with sodium pentobarbitol (35 mg/kg, i.v.). The dogs are respirated and the heart is exposed via a thoracotomy. Two pairs of plunge electrodes are placed in the mid-myocardium of the left ventricle for introduction of ventricular extra-stimuli. Two additional pairs of plunge electrodes are placed in the mid-myocardium within 1-2 mm of the ventricular pacing electrodes for recording local bipolar ventricular electrograms. Bipolar electrograms are recorded with the low and high frequency cutoffs set at 30 and 90 Hz, respectively. A pair of stainless steel electrodes are sutured onto the right atrial appendage for pacing the heart. The sinoatrial node is crushed, and the heart is paced at 150 beats per minute (5 msec duration, twice diastolic threshhold voltage or current). The local bipolar electrogram, as well as the Lead II ECG, is recorded continuously on a polygraph, and also acquired by a computer.

Ventricular effective refractory period (ERP) is determined by introduction of a single ventricular extrastimulus (4 msec duration, twice diastolic threshhold voltage or current) at progressively shorter delays after the right atrial stimulus, until a propagated ventricular depolarization is not seen in the Lead II ECG.

The ERP is defined as the longest interval between the ventricular extrastimulus that does not result in a propagated ventricular depolarization, and the preceding ventricular depolarization. The ERP is measured by the computer using the local bipolar electrogram from the site adjacent to ventricular stimulating electrodes. ERP's are determined at two sites in each heart and the values averaged. ERP's are initially determined under pretreatment conditions. Test compound is then administered either intravenously via a cannula placed in the right femoral vein, or intraduodenally via a cannula placed in the duodenum. ERP's are redetermined following the administration of that compound. The compounds of Formula I will increase the ERP.

The compounds of the present invention having Class III antiarrhythmic properties are useful for treating a variety of arrhythmic conditions of the heart. Representative examples of arrhythmic conditions which are amendable to treatment with the compounds of the present invention include supraventricular arrhythmias such as atrial tachycardia, atrial flutter, atrial fibrillation and paroxosysmal supraventricular tachycardia, and ventricular arrhythmias such as premature ventricular complexes, and life threatening ventricular arrhythmias such as ventricular tachycardia, or ventricular fibrillation. These compounds will also prevent recurrent episodes of the arrhythmias mentioned above.

The quantity of compound needed to either terminate an arrhythmic episode or prevent the occurrence of an arrhythmic episode (i.e., an antiarrhythmic quantity) will vary depending upon the route of administration, the patient, the severity of the patient's condition, the presence of other underlying disease states, and the particular compound utilized. However as a general guideline, if the compound is being administered orally, then it is preferably administered within a dosage range of from about 0.1 to about 100 mg/kg of patient body weight/day. Likewise, if the compound is being administered parenterally then it is preferably administered within a dosage range of from about 0.01 to about 10.0 mg/kg of patient body weight/day.

Repetitive daily administration may be desirable. Typically, the compounds will be administered from 1-4 times daily. They can also be administered as a continuous drip in critical care environments. The patient's response to the compound can be monitored via an EKG or any other technique conventionally used in the art.
As used in this application:
a) the term "arrhythmia" refers to any variation from the normal rhythm of the heart beat, and;
b) the term "antiarrhythmic" refers to a compound capable of either preventing or alleviating an arrhythmia.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations. In another embodiment, the compounds of Formula I can be tableted with conventional tablet bases such as lactose, sucrose, and cornstarch in combination with binders, such as acacia, cornstarch, or gelatin, disintegrating agents such as potato starch or algenic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration, the compounds may be dissolved in a physiologically acceptable pharmaceutical carrier and administered as either a solution or a suspension. Illustrative of suitable pharmaceutical carriers are water, n-saline, dextrose solutions, fructose solutions, ethanol, or oils of animal, vegetative, or synthetic origin. The pharmaceutical carrier may also contain preservatives, buffers, etc. as are known in the art.

The compounds of Formula I may be admixed with any inert carrier and utilized in laboratory assays in order to determine the concentration of the compounds within the urine, serum, etc. of the patient as is known in the art.

The following examples are presented in order to further illustrate the present invention. However, they should not be construed as limiting the scope of the invention in any manner.

### EXAMPLE I

This example demonstrates one of the N-alkylation reactions of Reaction Scheme I.

### A) N-[4[3-[4-(4-Fluorobenzoyl)-1-piperidinyl]1-oxopropyl]phenyl]methanesulfonamide monohydrochloride

A mixture of 4-fluorophenyl-4-piperidinyl methanone (3.30 g, 15.9 mmol) and N-[4-(3-chloro-1-oxopropyl)phenyl]methanesulfonamide was prepared in n-butanol (100 mL), treated with potassium bicarbonate (1.60 g, 16.0 mmol) and a catalytic amount of potassium iodide and refluxed for 18 hours. The mixture was cooled to room temperature and evaporated at reduced pressure onto neutral alumina. The resulting solid was put on top of a column of silica gel and eluted with acetone. The appropriate fractions were combined and evaporated to afford an orange oil. The oil was dissolved in ethyl acetate, dried (MgSO₄), filtered, and treated with a slight excess of HCl in EtOAc. The resulting solid was filtered and recrystallized from methanol to yield the desired product as tan crystals: mp 230-236°C (decomp.).

### EXAMPLE 2

This example demonstrates one of the N-alkylation reactions of Step A and one of the deprotection reactions of Step B.

### N-[4-[4-[4-(4-Fluorobenzoyl)-1-piperidinyl]-1-oxobutyl]phenyl]methanesulfonamide monohydrochloride

A mixture of 4-fluorophenyl-4-piperidinyl methanone (3.1 g, 15.0 mmol) and 2-(3-chloropropyl)-2-(4-methanesulfonamidophenyl)-1,3-dioxolane (4.70 g, 14.7 mmol) was prepared in n-butanol (50 mL), treated with potassium carbonate (2.00 g, 14.5 mmol) and a catalytic amount of potassium iodide, and refluxed for 18 hours. The cooled mixture was diluted with water and ethyl acetate. The layers were separated and the aqueous layer was extracted with ethyl acetate. The combined organic layers were washed with water, brine, dried (MgSO₄), filtered, and evaporated to an oil. The oil was dissolved in methanol (200 mL), treated with HCl (25 mL of a 10% solution in water), and stirred for 5 hours. The solution was concentrated, made basic with aqueous 5% sodium bicarbonate, and extracted twice with chloroform. The combined organic layers were washed with water, brine, dried (MgSO₄), filtered, and evaporated to a yellow solid. The solid was chromatographed on silica gel, eluting with acetone. The appropriate fractions were combined and evaporated to afford a yellow solid. The solid was dissolved in methanol and treated with a slight excess of HCl in methanol. The solution was concentrated and the resulting solid was recrystallized from methanol/butanone to yield the desired product as a tan powder: mp 205-210°C (decomp.).

### EXAMPLE 3

This example demonstrates the preparation of

### N-[4-[[(4-fluorobenzoyl)-1-piperidinyl]-acetyl]phenyl]methanesulfonamide hydrochloride

To a solution of 3.4 g (14.1 mmol) of 4-fluorobenzoyl-piperidine hydrochloride in 150 mL THF and 30 mL of water was added 5 g (59 mmol) of sodium bicarbonate followed by 3.5 g (14.1 mmol) of 2-chloro-4'-methanesulfonamidoacetophenone. After refluxing for 3.5 hours, the reaction was concentrated to 1/2 volume on a rotary evaporator. The mixture was extracted with chloroform, the organic layer dried with magnesium sulfate and concentrated. The resulting thick oil was treated with methanolic hydrogen chloride. The hydrochloride salt so obtained was recrystallized from methanol/2-butanone to give N-[4-[[(4-fluorobenzoyl)-1-piperidinyl]-acetyl]phenyl]methanesulfonamide hydrochloride as a white solid: mp 276-280°C (d).

### EXAMPLE 4

### N-[4-[[4-(2,3-Dimethoxybenzoyl)-1-piperidinyl]acetyl]phenyl]-methanesulfonamide monohydrochloride

A solution of 4-(2,3-dimethoxyphenyl)-4-piperidinylmethanone trifluoroacetate (2.0 g, 5.5 mmol), N-[4-(2-chloro-1-oxoethyl)phenyl]methanesulfonamide (1.19 g, 5.5 mmol) and KHCO₃ (1.1g, 11 mmol) was prepared in THF (75 mL) and H₂O (25 mL) and refluxed for 2 hours. The solution was concentrated and the resulting slurry was diluted with water and CH₂Cl₂. The partition was filtered through a glass filter and the resulting white solid was washed with CH₂Cl₂ and water. The white solid was dried overnight.

The layers of the filtrate were separated and the organic layer was washed with brine, dried (MgSO4) and evaporated to a yellow foam. The yellow foam and white powder were combined and chromatographed on silica gel eluting with 10% hexane in ethyl acetate. Evaporation of the appropriate fractions produced a white powder. The powder was dissolved in THF and treated with ethereal HCl to afford a white powder. The salt was recrystallized from methanol to afford N-[4-[[4-(2,3-dimethoxybenzoyl)-1-piperidinyl]acetyl]phenylmethane]sulfonamide monohydrochloride as fine white needles, m.p. 242-252°C (decomp.).
Anal. Calcd for C₂₃H₂₈N₂O₆S·HCl: C, 55.58; H, 5.88; N, 5.64.
Found: C, 55.45; H, 5.96; N, 5.75

### EXAMPLE 5

### N-[4-[[4-[(2,3-Dimethoxyphenyl)hydroxymethyl]-1-piperidinyl]acetyl]phenyl]methanesulfonamide monohydrochloride

To a stirred solution of α-(2,3-dimethoxyphenyl)-4-piperidine methanol (2.0 g, 8.0 mmol) in THF (60 mL) was added KHCO₃ (0.80 g, 8.0 mmol), then H₂O (6 mL), then N-[4-(2-chloro-1-oxoethyl)phenyl]methanesulfonamide (1.73 g, 8.0 mmol). After stirring overnight, the solution was concentrated, diluted with water, then extracted twice with CH₂Cl₂. The combined organic layers were dried (MgSO₄), and concentrated to 30 mL. This organic solution was chromatographed on silica gel, eluting with ethyl acetate.

The appropriate fractions were evaporated to a white foam. The foam was crystallized from isopropanol at reduced temperature and the resulting crystals were filtered under a nitrogen atmosphere and then washed with hexane. The resulting yellow solid was dried, producing N-[4-[[4-[(2,3-Dimethoxyphenyl)hydroxymethyl]-1-piperidinyl]acetyl]phenyl] methanesulfonamide monohydrochloride, m.p. 94-95°C. Anal. Calcd for C₂₃H₃₀N₂O₆S (462.56): C, 59.72; H, 6.54; N, 6.06. Found: C, 59.58; H, 6.68, N, 5.94.

Using the methodology above, but substituting the appropriate starting materials, N-[4-[[4-[(4-fluro phenyl)hydroxymethyl]-1-piperidinyl]acetyl]phenyl]methane sufonamide, mp 85-100°C, was obtained.

### EXAMPLE 6

### N-[4-[[4-(3,4-Diflourobenzoyl)-1-piperidinyl]acetyl]phenyl-methanesulfonamide monohydrochloride

A solution of 4-(3,4-difluorophenyl)-4-piperidinylmethanone hydrochloride (5.00 g, 19.1 mmol) and N-[4-(2-chloro-1-oxoethyl)phenyl]methanesulfonamide (5.2 g, 21.0 mmol) was prepared in THF (120 mL) and H₂O (20 mL), treated with KHCO₃ (4.00 g, 40 mmol) and stirred overnight for 5 days. The solution was concentrated, diluted with water, then extracted twice with CH₂Cl₂. The combined organic layers were dried (MgSO₄), and evaporated to an off-white powder which was dissolved in 300 mL of hot methanol. The solution was filtered and concentrated onto 40 g of neutral alumina. The alumina was put on top of a column of silica gel and eluted with 20% hexane in EtOAc.

The appropriate fractions were combined and treated with ethereal HCl. The resulting solid was recrystallized from methanol to afford 4.4 g of N-[4-[[4-(3,4-difluorobenzoyl)-1-piperidinyl]acetyl]phenyl]methanesulfonamide monohydrochloride as a pale yellow powder. m.p. >250°C (decomp.). Anal. calcd for C₂₁H₂₂F₂N₂O₄S·HCl: C, 53.33; H, 4.90; N, 5.92. Found: C, 53.28; H, 4.93; N, 5.82.

### EXAMPLE 7

### N-[4-[[4-(2,4,6-Trimethylbenzoyl)-1-piperidinyl]acetyl]phenyl]methanesulfonamide monohydrochloride

A solution of 4-(2,4,6-trimethylphenyl)-4-piperidinylmethanone monohydrochloride (2.5 g, 9.3 mmol) and N-[4-(2-chloro-1-oxoethyl)phenyl]methanesulfonamide (2.54 g, 10.3 mmol) was prepared in THF/H₂O (60/10), treated with KHCO₃ (2.0 g, 20 mmol), and stirred for 2 days. The solution was concentrated, diluted with water, then extracted twice with CHCl₃. The combined organic layers were dried (MgSO4), and evaporated to a tan foam. The foam was chromatographed on silica gel and eluted with 25% hexane in EtoAc.

The appropriate fractions were combined and evaporated to afford a yellow solid. The solid was dissolved in methanol and treated with HCl/methanol. The solution was concentrated and the resulting solid was recrystallized from methanol to afford N-[4-[[4-(2,4,6-trimethylbenzoyl)-1-piperidinyl]-acetyl]phenyl]methanesulfonamide monohydrochloride as white needles, m.p. >260°C (decomp.). Anal. calcd for C₂₄H₃₀N₂O₄S·HCl: C, 60.18; H, 6.52; N, 5.85. Found: C, 60.37; H, 6.67; N, 5.71.

### EXAMPLE 8

### N-[4-[[4-(4-Methylthio)benzoyl]-1-piperidinyl]acetyl]phenyl]-methanesulfonamide monohydrochloride

A solution of 4-methylthiophenyl-4-piperidinylmethanone monohydrochloride (5.00 g, 18.4 mmol), N-[4-(2-chloro-1-oxoethyl)phenyl]methanesulfonamide (5.00 g, 20.2 mmol), and KHCO₃ (3.8 g, 38 mmol) was prepared in aqueous THF (120 mL THF/20 mL H₂O) and stirred for 2 days. The solution was concentrated, diluted with water, then extracted twice with CHCl₃. The combined organic layers were dried (MgSO₄), and evaporated to afford a foam. The foam was chromatographed on silica gel and eluted with 3:1 EtoAc:hexane.

The appropriate fractions were combined and evaporated to afford a solid. The solid was dissolved in methanol and treated with HCl/methanol. The solution was concentrated and the resulting solid was recrystallized from methanol to afford N-[4-[[4-(4-(methylthio)benzoyl]-1-piperidinyl]acetyl]phenyl]-methanesulfonamide monohydrochloride as light rose colored flakes, m.p. >260°C (decomp.). Anal. Calcd for C₂₂H₂₆N₂O₄S₂·HCl. C, 54.70; H, 5.63; N, 5.80. Found: C, 54.81; H, 5.68; N, 5.72.

Utilizing the methodology of Example 8 but substituting the appropriate starting materials the following compounds may be prepared:
a) N-[4[[4-(4-chlorobenzoyl)-1-piperidinyl]acetyl] phenyl]methanesulfonamide hydrochloride m.p. >250°C (dec.);
b) N-[4[[4-(4-acetamidobenzoyl)-1-piperidinyl]acetyl]phenyl]methanesulfonamide m.p. 200-210 (dec.);
c) N-[4[[4(4-aminobenzoyl)-1-piperidinyl]acetyl] phenyl]methanesulfonamide hydrochloride m.p. 230-250°C;
d) N-[4[[4-(3,4-difluorobenzoyl)-1-piperidinyl]-1-oxopropyl] phenyl]methanesulfonamide m.p. > 137-138°C (dec.);
e) N-[4[[4-(2-ethoxybenzoyl)-1-piperidinyl]acetyl] phenyl]methanesulfonamide m.p. 155-156°C (dec.);
f) N-[4[[3-(2-methoxybenzoyl)-1-piperidinyl]acetyl] phenyl]methanesulfonamide hydrochloride m.p. 245-247°C (dec.);
g) N-[4[[3-(4-fluorobenzoyl)-1-piperidinyl]acetyl] phenyl]methanesulfonamide;
h) N-[4[[3-(3,4-difluorobenzoyl)-1-piperidinyl]acetyl] phenyl]methanesulfonamide;
i) N-[4[[3-(4-chlorobenzoyl)-1-piperidinyl]acetyl] phenyl]methanesulfonamide;
j) N-[4[[3-(4-methylthiobenzoyl)-1-piperidinyl]acetyl] phenyl]methanesulfonamide.

## Claims

1. A compound of the formula: 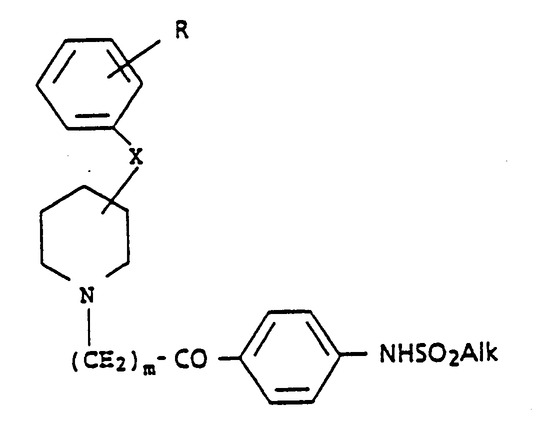 in which R is represented by hydrogen, halogen, C₁₋₄ alkyl, C₁₋₄ alkoxy, CF₃, SR₁, or OH wherein the phenyl ring may contain up to 3 non-hydrogen substituents; R₁ is hydrogen or C₁₋₄ alkyl; X is represented by CO or CHOH; m is an integer from 1-3; and Alk is a C₁₋₄ alkyl; and the pharmaceutically acceptable addition salts thereof.

2. A compound according to claim 1 wherein X is CHOH.

3. A compound according to claim 1 wherein X is CO.

4. A compound according to claim 3 wherein m is 1.

5. A compound according to claim 3 wherein m is 2.

6. A compound according to claim 5 wherein Alk is methyl.

7. A compound according to claim 1 wherein said compound is
N-[4-[[4-(2,3-dimethoxybenzoyl)-1-piperidinyl]acetyl]phenyl]methanesulfonamide,
N-[4-[[4-(3,4-difluorobenzoyl)-1-piperidinyl]acetyl]phenyl]-methanesulfonamide,
N-[4-[[4-(2,4,6-trimethylbenzoyl)-1-piperidinyl]acetyl]phenyl]methanesulfonamide,
N-[4-[[4-[4-(methylthio)benzoyl]-1-piperidinyl]acetyl]phenyl]methanesulfonamide,
N-[4-[[4-(2-ethoxybenzoyl)-1-piperidinyl]acetyl]phenyl]methanesulfonamide,
N-[4-[[4-[(2,3-dimethoxyphenyl)hydroxymethyl]-1-piperidinyl]acetyl]phenyl]methanesulfonamide,
N-[4-[[4-[(4-fluorophenyl)hydroxymethyl]-1-piperidinyl]acetyl]phenyl]methanesulfonamide,
N-[4-[4-[4-(4-fluorbenzoyl)-1-piperidinyl]-1-oxobutyl]phenyl]methanesulfonamide monohydrochloride,
N-[4-[3-[4-(4-fluorobenzoyl)-1-piperidinyl]-1-oxopropyl]-phenyl]methanesulfonamide monohydrochloride,
N-[4-[3-[4-(4-fluorobenzoyl)-1-piperidinyl]-1-oxopropyl]-phenyl]methanesulfonamide,
N-[4-[[(4-chlorobenzoyl)-1-piperidinyl]acetyl]-phenyl]methanesulfonamide,
N-[4-[[(4-chlorobenzoyl)-1-piperidinyl]oxopropyl]phenyl]methanesulfonamide,
N-[4-[[4-[(3,4-difluorobenzoyl)-1-piperidinyl]-1-oxopropyl]-phenyl]methanesulfonamide,
N-[4-[[4-[(3,4-difluorophenyl)hydroxymethyl]-1-piperidinyl]acetyl]phenyl]methanesulfonamide,
N-[4-[3-[4-[(3,4-difluorophenyl)hydroxymethyl]-1-piperidinyl]oxopropyl]phenyl]methanesulfonamide,
N-[4-[[3-(2-methoxybenzoyl)-1-piperidinyl]acetyl]phenyl]methanesulfonamide hydrochloride,
N-[4-[[3-(4-fluorobenzoyl)-1-piperidinyl]acetyl]phenyl]methanesulfonamide,
N-[4-[[3-(3,4-difluorobenzoyl)-1-piperidinyl]acetyl]phenyl]methanesulfonamide or
N-[4-[[3-(4-chlorobenzoyl)-1-piperidinyl]acetyl]phenyl]methanesulfonamide.

8. A compound according to any of claims 1, 2 or 3 in which X is bonded to the 4-position of the piperidine ring.

9. A pharmaceutical composition comprising an effective amount of a compound according to any of claims 1-7 in admixture with a pharmaceutically acceptable carrier.

10. Use of a compound according to any of claims 1-7 as a medicine.

11. Use of a compound according to any of claims 1-7 in the manufacture of a medicament for cardiac arrhythmias.

12. A process for preparing a compound as defined in claims 1-8, wherein a compound of formula: 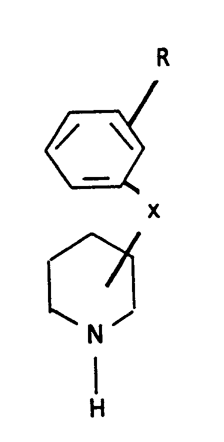 is reacted with a compound of formula 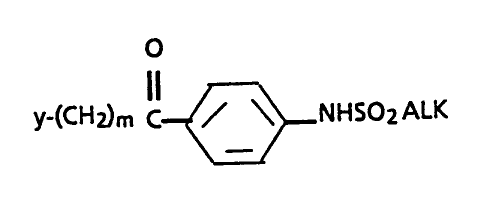 in which y is represented by a leaving group and R, X, m and Alk are as defined in claim 1.

13. A process as in claim 12 wherein the reaction is carried out in the presence of a solvent and in the presence of a base.

14. A process as in claim 13 wherein y is represented by halogen, or tosylate.

15. A method for the preparation of a pharmaceutical composition comprising combining a compound according to any one of claims 1-7 with a pharmaceutical carrier.

16. A method according to claim 15 wherein the pharmaceutical composition is for treating cardiac arrhythmias.

## Patentansprüche

1. Verbindung der Formel 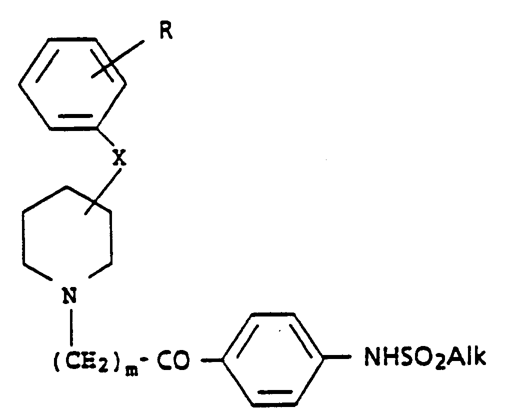 in der R ein Wasserstoffatom, ein Halogenatom, einen C₁₋₄-Alkyl- oder C₁₋₄-Alkoxyrest, CF₃, SR₁ oder OH bedeutet, wobei der Phenylring bis zu drei Substituenten enthalten kann, die kein Wasserstoffatom bedeuten, R₁ ein Wasserstoffatom oder einen C₁₋₄-Alkylrest bedeutet, X CO oder CHOH bedeutet, m eine ganze Zahl von 1 bis 3 bedeutet und Alk einen C₁₋₄-Alkylrest bedeutet und die pharmazeutisch verträglichen Säureadditionssalze davon.

2. Verbindung nach Anspruch 1, in der X CHOH bedeutet.

3. Verbindung nach Anspruch 1, in der X CO bedeutet.

4. Verbindung nach Anspruch 3, in der m 1 bedeutet.

5. Verbindung nach Anspruch 3, in der m 2 bedeutet.

6. Verbindung nach Anspruch 5, in der Alk eine Methylgruppe bedeutet.

7. Verbindung nach Anspruch 1, nämlich
N-[4-[[4-(2,3-Dimethoxybenzoyl)-1-piperidinyl]acetyl]phenyl]methansulfonamid,
N-[4-[[4-(3,4-Difluorbenzoyl)-1-piperidinyl]acetyl]phenyl]methansulfonamid,
N-[4-[[4-(2,4,6-Trimethylbenzoyl)-1-piperidinyl]acetyl]phenyl]methansulfonamid,
N-[4-[[4-[4-(Methylthio)benzoyl]-1-piperidinyl]acetyl]phenyl]methansulfonamid,
N-[4-[[4-(2-Ethoxybenzoyl)-1-piperidinyl]acetyl]phenyl]methansulfonamid,
N-[4-[[4-[(2,3-Dimethoxyphenyl)hydroxymethyl]-1-piperidinyl]acetyl]phenyl]methansulfonamid,
N-[4-[[4-[(4-Fluorphenyl)hydroxymethyl]-1-piperidinyl]acetyl]phenyl]methansulfonamid,
N-[4-[4-[4-(4-Fluorbenzoyl)-1-piperidinyl]-1-oxobutyl]phenyl]methansulfonamid-Monohydrochlorid,
N-[4-[3-[4-(4-Fluorbenzoyl)-1-piperidinyl]-1-oxopropyl]phenyl]methansulfonamid-Monohydrochlorid,
N-[4-[3-[4-(4-Fluorbenzoyl)-1-piperidinyl]-1-oxopropyl]phenyl]methansulfonamid,
N-[4-[[(4-Chlorbenzoyl)-1-piperidinyl]acetyl]phenyl]methansulfonamid,
N-[4-[[(4-Chlorbenzoyl)-1-piperidinyl]oxopropyl]phenyl]methansulfonamid,
N-[4-[[4-[(3,4-Difluorbenzoyl)-1-piperidinyl]-1-oxopropyl]phenyl]methansulfonamid,
N-[4-[[4-[(3,4-Difluorbenzoyl)hydroxymethyl]-1-piperidinyl]acetyl]phenyl]methansulfonamid,
N-[4-[3-[4-[(3,4-Difluorphenyl)hydroxymethyl]-1-piperidinyl]oxopropyl]phenyl]methansulfonamid,
N-[4-[[3-(2-Methoxybenzoyl)-1-piperidinyl]acetyl]phenyl]methansulfonamid-Hydrochlorid,
N-[4-[[3-(4-Fluorbenzoyl)-1-piperidinyl]acetyl]phenyl]methansulfonamid,
N-[4-[[3-(3,4-Difluorbenzoyl)-1-piperidinyl]acetyl]phenyl]methansulfonamid oder
N-[4-[[3-(4-Chlorbenzoyl)-1-piperidinyl]acetyl]phenyl]methansulfonamid.

8. Verbindung nach einem der Ansprüche 1, 2 oder 3, in der X in 4-Position an den Piperidinring gebunden ist.

9. Arzneimittel, umfassend eine wirksame Menge einer Verbindung nach einem der Ansprüche 1 bis 7 im Gemisch mit einem pharmazeutisch verträglichen Träger.

10. Verwendung einer Verbindung nach einem der Ansprüche 1 bis 7 als Arzneimittel.

11. Verwendung einer Verbindung nach einem der Ansprüche 1 bis 7 zur Herstellung eines Arzneimittels gegen kardiale Arrhythmie.

12. Verfahren zur Herstellung einer Verbindung nach einem der Ansprüche 1 bis 8, wobei eine Verbindung der Formel 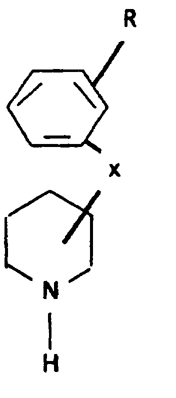 mit einer Verbindung der Formel 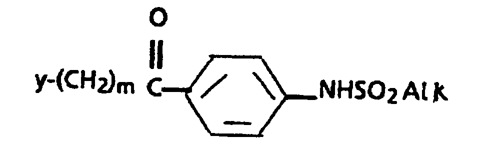 umgesetzt wird, wobei y eine Abgangsgruppe darstellt und R, X, m und Alk wie in Anspruch 1 definiert sind.

13. Verfahren nach Anspruch 12, wobei die Umsetzung in Gegenwart eines Lösungsmittels und in Gegenwart einer Base ausgeführt wird.

14. Verfahren nach Anspruch 13, wobei y ein Halogenatom oder eine Tosylatgruppe darstellt.

15. Verfahren zur Herstellung eines Arzneimittels, umfassend die Vereinigung einer Verbindung nach einem der Ansprüche 1 bis 7 mit einem pharmazeutischen Träger.

16. Verfahren nach Anspruch 15, wobei das Arzneimittel für die Behandlung von kardialer Arrhythmie ist.

## Revendications

1. Composé répondant à la formule : 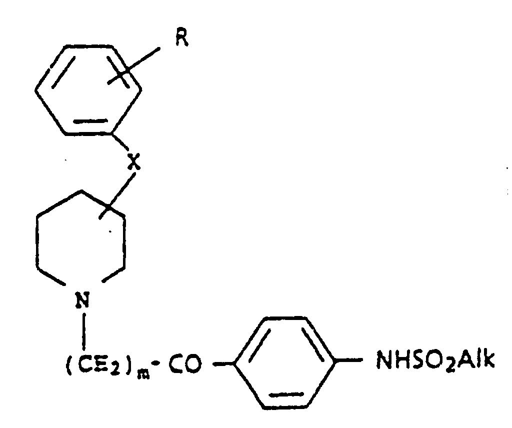 dans laquelle R représente un atome d'hydrogène, d'halogène, un groupe alkyle en C₁₋₄, alcoxy en C₁₋₄, CF₃, SR₁, ou OH dans lequel le cycle phényle peut contenir jusqu'à 3 substituants qui ne sont pas des atomes d'hydrogène ; R₁ représente un atome d'hydrogène ou un groupe alkyle en C₁₋₄ ; X représente CO ou CHOH ; m est un nombre entier compris entre 1 et 3 ; et Alk est un groupe alkyle en C₁₋₄ ; et ses sels par addition acceptables du point de vue pharmaceutique.

2. Composé conforme à la revendication 1, dans lequel X représente CHOH.

3. Composé conforme à la revendication 1, dans lequel X représente CO.

4. Composé conforme à la revendication 3, dans lequel m vaut 1.

5. Composé conforme à la revendication 3, dans lequel m vaut 2.

6. Composé conforme à la revendication 5, dans lequel Alk représente un groupe méthyle.

7. Composé conforme à la revendication 1, ledit composé étant
le N-[4-[[4-(2,3-diméthoxybenzoyl)-1-piperidinyl]acétyl]phényl]méthanesulfonamide,
le N-[4-[[4-(3,4-difluorobenzoyl)-1-piperidinyl]acétyl]phényl]méthanesulfonamide,
le N-[4-[[4-(2,4,6-triméthylbenzoyl)-1-piperidinyl]acétyl]phényl]méthanesulfonamide,
le N-[4-[[4-[4-(méthylthio)benzoyl]-1-piperidinyl]acétyl]phényl]méthanesulfonamide,
le N-[4-[[4-(2-éthoxybenzoyl)-1-piperidinyl]acétyl]phényl]méthanesulfonamide,
le N-[4-[[4-[(2,3-diméthoxyphényl)hydroxyméthyl]-1-piperidinyl]acétyl]phényl]méthanesulfonamide,
le N-[4-[[4-[(4-fluorophényl)hydroxyméthyl]-1-piperidinyl]acétyl]phényl]méthanesulfonamide,
le monochlorhydrate de N-[4-[4-[4-(4-fluorobenzoyl)-1-piperidinyl]-1-oxobutyl]phényl]méthanesulfonamide,
le monochlorhydrate de N-[4-[3-[4-(4-fluorobenzoyl)-1-piperidinyl]-1-oxopropyl]phényl]méthanesulfonamide,
le N-[4-[3-[4-(4-fluorobenzoyl)-1-piperidinyl]-1-oxopropyl]phényl]méthanesulfonamide,
le N-[4-[[(4-chlorobenzoyl)-1-piperidinyl]acétyl]phényl]méthanesulfonamide,
le N-[4-[[(4-chlorobenzoyl)-1-piperidinyl]oxopropyl]phényl]méthanesulfonamide,
le N-[4-[[4-[(3,4-difluorobenzoyl)-1-piperidinyl]-1-oxopropyl] phényl]méthanesulfonamide,
le N-[4-[[4-[(3,4-difluorophényl)hydroxyméthyl]-1-piperidinyl]acétyl]phényl]méthanesulfonamide,
le N-[4-[3-[4-[(3,4-difluorophényl)hydroxyméthyl]-1-piperidinyl] oxopropyl] phényl]méthanesulfonamide,
le chlorhydrate de N-[4-[[3-(2-méthoxybenzoyl)-1-piperidinyl]acétyl]phényl]méthanesulfonamide,
N-[4-[[3-(4-fluorobenzoyl)-1-piperidinyl]acétyl]phényl]méthanesulfonamide,
le N-[4-[[3-(3,4-difluorobenzoyl)-1-piperidinyl]acétyl]phényl]méthanesulfonamide ou
le N-[4-[[3-(4-chlorobenzoyl)-1-piperidinyl]acétyl]phényl]méthanesulfonamide.

8. Composé conforme à l'une quelconque des revendications 1, 2 ou 3 dans lequel X est lié à la position 4 du cycle piperidine.

9. Composition pharmaceutique comprenant une quantité efficace d'un composé conforme à l'une quelconque des revendications 1 à 7, mélangé à un véhicule acceptable du point de vue pharmaceutique.

10. Utilisation d'un composé conforme à l'une quelconque des revendications 1 à 7 comme médicament.

11. Utilisation d'un composé conforme à l'une quelconque des revendications 1 à 7 dans la fabrication d'un médicament destiné au traitement des arythmies cardiaques.

12. Procédé pour la préparation d'un composé tel que défini dans les revendications 1 à 8, dans lequel on fait réagir un composé répondant à la formule : 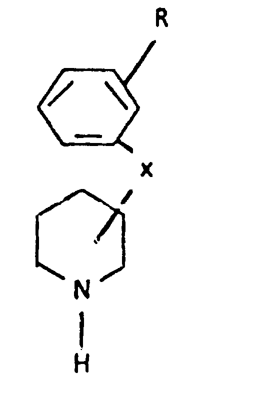 avec un composé répondant à la formule : 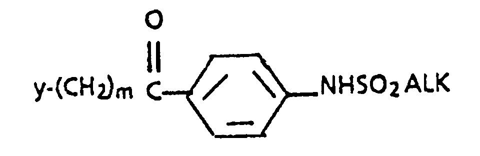 dans laquelle y représente un groupe de départ et R, X, m et Alk sont tels que définis dans la revendication 1.

13. Procédé de la revendication 12, dans lequel la réaction est effectuée en présence d'un solvant et en présence d'une base.

14. Procédé de la revendication 13, dans lequel y représente un atome d'halogène ou un tosylate.

15. Procédé de préparation d'une composition pharmaceutique, comprenant la combinaison d'un composé conforme à l'une quelconque des revendications de 1 à 7 avec un véhicule pharmaceutique.

16. Procédé conforme à la revendication 15, dans laquelle la composition pharmaceutique est destinée au traitement des arythmies cardiaques.
